# EUROPEAN PATENT SPECIFICATION

(11) **EP 3 698 813 B1**
(45) Date of publication and mention of the grant of the patent: **03.05.2023**
(21) Application number: 18884149.8
(22) Date of filing: 29.11.2018
(51) Int. Cl.: A61L 27/00, A61B 17/56

(54) **ARTIFICIAL BONE**
KÜNSTLICHER KNOCHEN
OS ARTIFICIEL

(30) Priority: 01.12.2017 HK 17112754
(43) Date of publication of application: 26.08.2020
(73) Proprietor: Koln 3D Technology (Medical) Limited, Shatin, N.T., Hong Kong (CN)
(72) Inventor: YAU, Wing Fung Edmond, Shatin, N.T., Hong Kong (CN); MAK, Sze Yi, Shatin, N.T., Hong Kong (CN); TSE, Lung Fung, Shatin, N.T., Hong Kong (CN)
(74) Representative: Loo, Chi Ching
(86) International application number: PCT/IB2018/059433
(87) International publication number: WO 2019/106582

(56) References cited:
- WO-A1-2006/078662
- WO-A1-2011/138646
- CN-A- 101 108 145
- CN-A- 102 824 207
- CN-U- 202 146 350
- HK-A2- 1 246 069
- US-A1- 2017 165 077
- US-B1- 8 956 394

## Description

### Technical Field

The invention relates to bionics, in particular to an artificial bone.

### Background Art

With the development of science and technology, life span of human is increasing continuously. Nonetheless, the body's function may decline with age or because of disease. Bionic organs are urgently needed to replace native ones. WO 2011/138646 A1 describes an integrated prosthetic element useable for bone implant operations or as a bone filler or replacement. Patent document WO 2006/078662 A1 discloses an artificial bone made of two cylindrical portions and an intermediate portion, as in the preamble of claim 1 of the present invention.

### Summary of the Invention

### Solution to the Problems

In order to solve above existing technical problems, the invention provides an artificial bone as claimed in claim 1.

Further optional improvements are set forth in claims 2 to 5.

### Advantageous result of the invention

In comparison with existing technologies, the advantageous result of the invention is: use artificial bone to replace original bone, set supporting pillar and 3D porous scaffold structure in the meantime, the supporting pillar gets into coupling end which connected to original bone to work as support and fixing. Set the 3D porous scaffold structure on the periphery of the coupling end which connected to original bone, to provide space for the adhesion and growth of bone cells, and to make the connection between artificial bone and original bone more stable.

### Brief Description of the Drawings

Fig.1

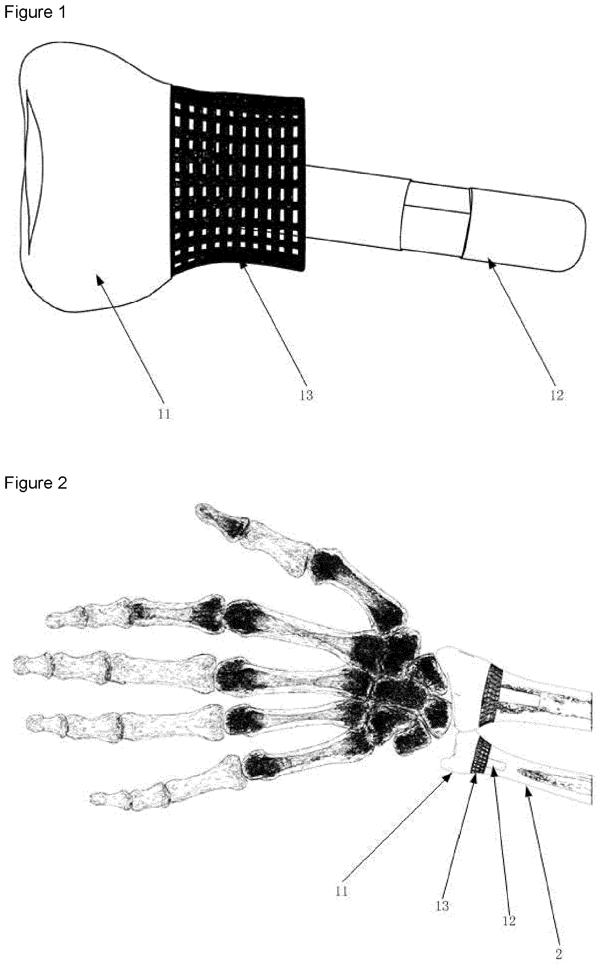

   Fig.1 is the structural diagram of artificial bone of the invention;
Fig.2
   Fig.2 is an embodiment diagram of said artificial bone;
Fig.3

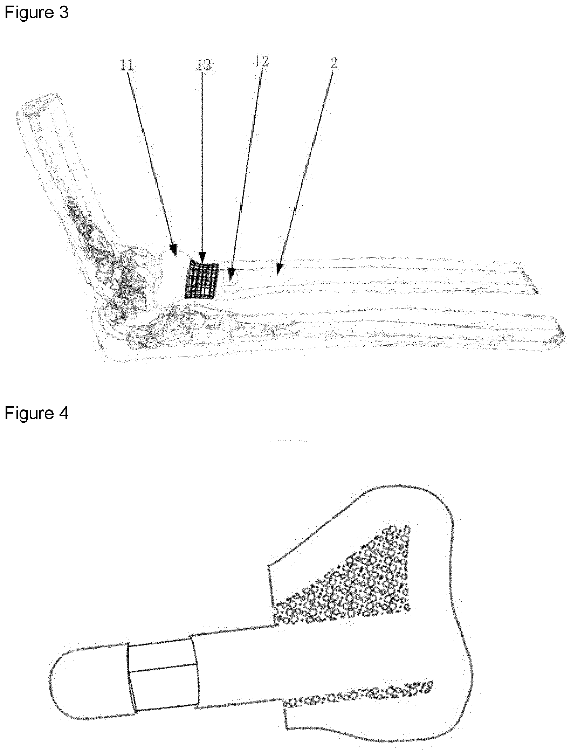

   Fig.3 is another embodiment diagram of said artificial bone;
Fig.4
   Fig.4 is the structural diagram of the first embodiment of artificial bone in the invention;
Fig.5

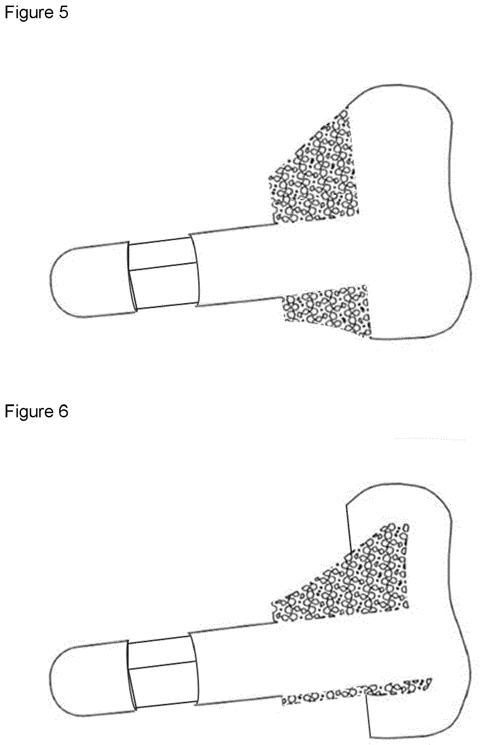

   Fig.5 is the structural diagram of the second embodiment of artificial bone in the invention;
Fig.6
   Fig.6 is the structural diagram of the third embodiment of artificial bone in the invention;
Fig.7

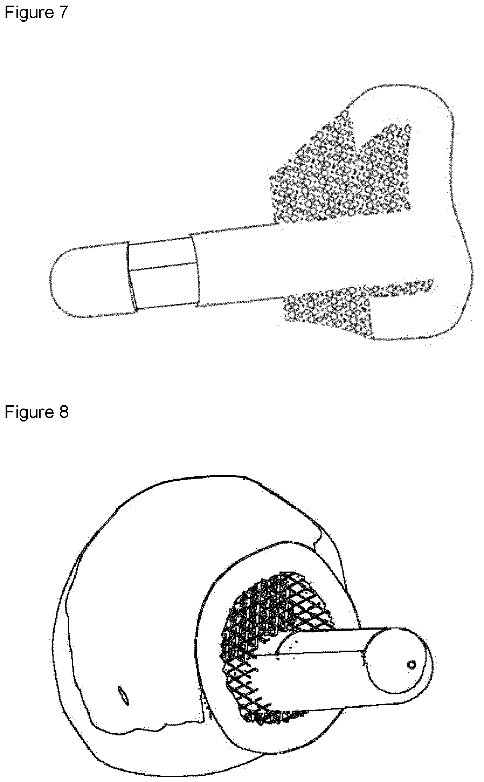

   Fig.7 is the structural diagram of the fourth embodiment of artificial bone in the invention;
Fig.8
   Fig.8 is the 3D view of the first embodiment of artificial bone in the invention;
Fig.9

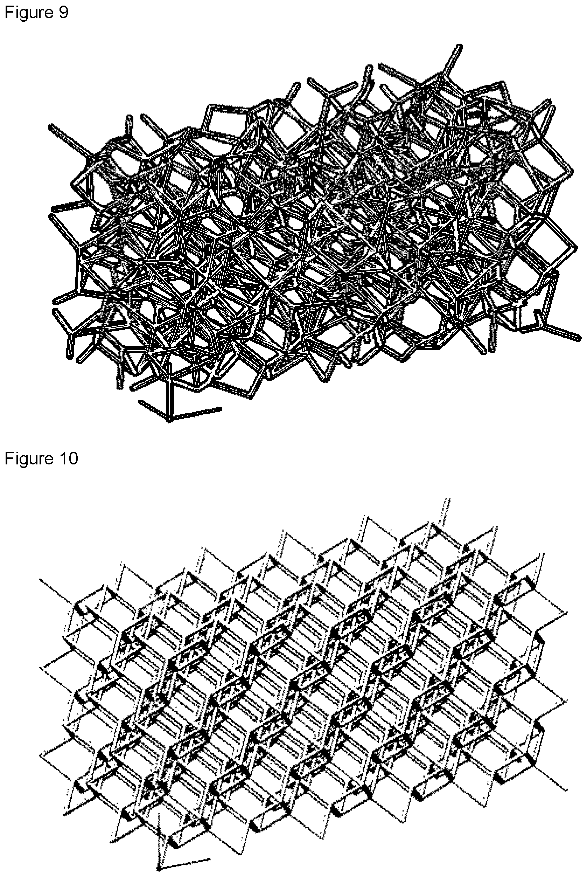

   Fig.9 is the structural diagram of 3D porous scaffold structure of artificial bone in an embodiment in the invention;
Fig.10
   Fig.10 is the structural diagram of 3D porous scaffold structure of artificial bone in another embodiment in the invention.

Bionic bone 11; supporting pillar 12; 3D porous scaffold structure 13; original bone 2.

### Detailed Description of the Invention

Below further describe the invention combining with drawings and embodiment.

As shown in Fig.1-3, an artificial bone, comprising bionic bone, supporting pillar and 3D porous scaffold structure; supporting pillar and 3D porous scaffold structure are connected to bionic bone, 3D porous scaffold structure is set to cover the supporting pillar. Use the artificial bone to replace original bone, in the meantime, set the supporting pillar and 3D porous scaffold structure to strengthen the connection between artificial bone and original bone and promote the subsequent growth of bone cells.

Supporting pillar of the utility model is two columns and the prism between two columns, cross section of said prism is rectangle, it can be the fixing part when burnishing, grinding or polishing the bionic bone, also prevent rotation and shift between supporting pillar and original bone. Specifically, cross section area of prism is smaller than the cross section area of the column. Said supporting pillar can get into coupling end which connected to original bone, 3D porous scaffold structure is set to cover the coupling end which connected to original bone.

Shape and size of the pore of said 3D porous scaffold structure in the utility model are changed according to actual need or patient's actual situation.

Bionic bone in the utility model is made according to 3D data of replaced original bone, supporting pillar is made according to inclination of original bone. There are several manufacture methods of artificial bone: in the first method, bionic bone, supporting pillar, and 3D porous scaffold structure are an integral structure. The integral structure is directly printed out by 3D metal printer using cobalt-chromium alloy or titanium alloy. In the second method, bionic bone, supporting pillar, and 3D porous scaffold structure are an integral structure, which is directly printed out by 3D metal printer using titanium alloy, then plated with cobalt-chromium alloy on the surface. In the third method, supporting pillar and 3D porous scaffold structure are connected to bionic bone respectively through screw thread, i.e. use cobalt-chromium alloy to print out the bionic bone by 3D metal printer at first, then use titanium alloy to print out supporting pillar and 3D porous scaffold structure by 3D metal printer, after that assemble the supporting pillar and 3D porous scaffold structure with bionic bone respectively through threaded connection. In the fourth method, supporting pillar and 3D porous scaffold structure are connected to bionic bone by sintering, i.e. use cobalt-chromium alloy to print out the bionic bone by 3D metal printer at first, then use titanium alloy to print out supporting pillar and 3D porous scaffold structure by 3D metal printer, after that sinter the supporting pillar and 3D porous scaffold structure to bionic bone respectively.

Above are the further detailed description of the invention combining with preferable embodiment, but cannot limit the embodiment of invention to these only.

## Claims

1. An artificial bone comprising:
a bionic bone (11) dimensioned according to 3D data of a portion of an original bone (2) to be replaced;
a supporting pillar (12) dimensioned according to an inclination of the original bone (2), for connecting the bionic bone (11) to a remaining portion of the original bone (2); and
a 3D porous scaffold structure (13);
wherein said supporting pillar (12) and said 3D porous scaffold structure (13) are connected to said bionic bone (11), said 3D porous scaffold structure (13) covers said supporting pillar (12),
wherein said supporting pillar (12) is formed of two cylindrical-shaped portions and **characterized in that** a cuboidal-shaped portion extends between the two cylindrical-shaped portions.

2. The artificial bone of Claim 1, wherein, a cross sectional area of said cuboidal-shaped portion is smaller than a cross sectional area of each said cylindrical-shaped portion.

3. The artificial bone of Claim 1, wherein, said supporting pillar (12) is connectable into a coupling end of the original bone (2), such that in use said 3D porous scaffold structure (13) covers the coupling end of the original bone (2).

4. The artificial bone of Claim 1, wherein, shape and size of pores of said 3D porous scaffold structure (13) are dimensioned according to actual need or patient's actual situation.

5. The artificial bone of any of Claim 1-4, wherein, said bionic bone (11) and said supporting pillar (12), said 3D porous scaffold structure (13) are an integral structure; or, said supporting pillar (12) and said 3D porous scaffold structure (13) are connected to said bionic bone (11) through screw thread, respectively; or, said supporting pillar (12) and said 3D porous scaffold structure (13) are connected to said bionic bone (11) by sintering, respectively.

## Patentansprüche

1. Künstlicher Knochen, umfassend:
einen bionischen Knochen (11), der gemäß 3D-Daten eines Abschnitts eines ursprünglichen Knochens (2), der ersetzt werden soll, bemessen ist;
eine Stützsäule (12), die gemäß einer Neigung des ursprünglichen Knochens (2) bemessen ist, zum Verbinden des bionischen Knochens (11) mit einem verbleibenden Abschnitt des ursprünglichen Knochens (2); und
eine poröse 3D-Gerüststruktur (13);
wobei die Stützsäule (12) und die poröse 3D-Gerüststruktur (13) mit dem bionischen Knochen (11) verbunden sind, wobei die poröse 3D-Gerüststruktur (13) die Stützsäule (12) bedeckt,
wobei die Stützsäule (12) aus zwei zylinderförmigen Abschnitten ausgebildet ist und **dadurch gekennzeichnet ist, dass** sich ein quaderförmiger Abschnitt zwischen den zwei zylinderförmigen Abschnitten erstreckt.

2. Künstlicher Knochen nach Anspruch 1, wobei eine Querschnittsfläche des quaderförmigen Abschnitts kleiner als eine Querschnittsfläche jedes zylinderförmigen Abschnitts ist.

3. Künstlicher Knochen nach Anspruch 1, wobei die Stützsäule (12) in ein Kopplungsende des ursprünglichen Knochens (2) derart verbindbar ist, dass die poröse 3D-Gerüststruktur (13) in Verwendung das Kopplungsende des ursprünglichen Knochens (2) bedeckt.

4. Künstlicher Knochen nach Anspruch 1, wobei Form und Größe von Poren der porösen 3D-Gerüststruktur (13) gemäß einem tatsächlichen Bedarf oder einer tatsächlichen Situation des Patienten bemessen sind.

5. Künstlicher Knochen nach einem der Ansprüche 1 bis 4, wobei der bionische Knochen (11) und die Stützsäule (12), die poröse 3D-Gerüststruktur (13) eine integrale Struktur sind; oder die Stützsäule (12) und die poröse 3D-Gerüststruktur (13) jeweils durch ein Schraubengewinde mit dem bionischen Knochen (11) verbunden sind; oder die Stützsäule (12) und die poröse 3D-Gerüststruktur (13) jeweils durch ein Sintern mit dem bionischen Knochen (11) verbunden sind.

## Revendications

1. Os artificiel comprenant :
un os bionique (11) dimensionné selon des données 3D d'une partie d'un os d'origine (2) à remplacer ;
un montant de support (12) dimensionné selon une inclinaison de l'os d'origine (2), pour relier l'os bionique (11) à une partie restante de l'os d'origine (2) ; et
une structure d'échafaudage poreuse 3D (13) ;
ledit montant de support (12) et ladite structure d'échafaudage poreuse 3D (13) étant reliés audit os bionique (11), ladite structure d'échafaudage poreuse 3D (13) recouvrant ledit montant de support (12),
ledit montant de support (12) étant formé de deux parties de forme cylindrique et **caractérisé en ce qu'**une partie de forme cubique s'étend entre les deux parties de forme cylindrique.

2. Os artificiel selon la revendication 1, dans lequel une aire de section transversale de ladite partie de forme cubique est plus petite qu'une aire de section transversale de chaque dite partie de forme cylindrique.

3. Os artificiel selon la revendication 1, dans lequel ledit montant de support (12) peut être relié à une extrémité d'accouplement de l'os d'origine (2), de sorte que, lors de son utilisation, ladite structure d'échafaudage poreuse 3D (13) recouvre l'extrémité d'accouplement de l'os d'origine (2).

4. Os artificiel selon la revendication 1, dans lequel la forme et la taille des pores de ladite structure d'échafaudage poreuse 3D (13) sont dimensionnées en fonction du besoin réel ou de la situation réelle du patient.

5. Os artificiel selon l'une quelconque des revendications 1 à 4, dans lequel ledit os bionique (11) et ledit montant de support (12), ladite structure d'échafaudage poreuse 3D (13) sont une structure intégrale ; ou ledit montant de support (12) et ladite structure d'échafaudage poreuse 3D (13) sont reliés audit os bionique (11) par un filet de vis, respectivement ; ou ledit montant de support (12) et ladite structure d'échafaudage poreuse 3D (13) sont reliés audit os bionique (11) par frittage, respectivement.
